# EUROPEAN PATENT APPLICATION

(11) **EP 2 253 276 A1**
(43) Date of publication of application: **24.11.2010**
(21) Application number: 10250945.2
(22) Date of filing: 18.05.2010
(51) Int. Cl.: A61B 17/00

(54) **Wound closure system**

(30) Priority: 19.05.2009 US 179437 P; 14.04.2010 US 759799
(71) Applicant: Tyco Healthcare Group LP, North Haven, CT 06473 (US)
(72) Inventor: Fischvogt, Gregory, Hamden, CT 06514 (US)
(74) Representative: Soames, Candida Jane

(57) **Abstract**

Accordingly, a wound closure system for closing a puncture wound is provided. The system includes a housing for maintaining a vacuum about a wound, a sealing assembly including a working instrument having a distal end configured to be inserted through the housing and into the wound, wherein the working instrument is further configured to close and seal the wound as the working instrument is withdrawn therefrom.

## Description

### CROSS REFERENCE TO RELATED APPLICATION

The present application claims the benefit of and priority to U.S. Provisional Application Serial No. 61/179,437 filed on May 19, 2009, the entire contents of which are incorporated herein by reference.

### BACKGROUND

### Technical Field

The present disclosure relates to access assemblies and means for accessing a body cavity. More particularly, the present disclosure relates to a system and method for sealing a puncture wound created by an access assembly.

### Background of Related Art

Surgical access devices, including trocars and port assemblies, are known, as are myriad of procedures that may be performed through these devices. Typically, an access device includes a housing and a cannula extending from the housing. The housing may include valves, seals and other mechanisms for directing an instrument into a patient. Cannulas typically are configured to pass through the skin of the patient into a body cavity, either through the use of a bladed tip or through a premade incision.

Upon completion of a closed procedure, the one or more access devices used to access the body cavity of the patient are removed, thus creating one or more puncture wounds. The size of the puncture wound may vary depending on the size of the cannula used to access the body cavity. Certain procedures require a larger passageway into the body cavity in which to complete the procedure. Sealing the puncture wounds using conventional methods, i.e. staples or sutures, is time consuming, require removal once the wound is healed, and may result in unnecessary scarring.

Therefore, it would be beneficial to have a system and method of closing a wound that does not require the use of mechanical fasteners.

### SUMMARY

Accordingly, a wound closure system for closing a puncture wound is provided. The system includes a housing for maintaining a vacuum about a wound, a sealing assembly including a working instrument having a distal end configured to be inserted through the housing and into the wound, wherein the working instrument is further configured to close and seal the wound as the working instrument is withdrawn therefrom.

The housing of the wound closure system may define a hemispherical member. The housing may be transparent. In one embodiment, the system further includes an adhesive supply source operably connected to the working instrument and a vacuum source operably connected to the working instrument. In an alternate embodiment, the system includes an electrosurgical generator operably connect to the working instrument. The working instrument may include at least a first switch for selectively activating a first function of the sealing assembly and may include a second switch for selectively activating a second function of the sealing assembly. The working instrument may be configured to selectively dispense an adhesive and/or selectively supply a vacuum to the housing. The working instrument may also or instead be configured to selectively fuse tissue. The system may further include an absorbent film for positioning adjacent the wound.

Also provided is a method of closing a wound. The method includes the steps of providing a wound closure system including a housing and a sealing assembly including a working instrument, placing the housing in a sealed relationship over the wound, inserting a distal end of the working instrument through the housing and into the wound, applying a vacuum to the housing, activating the working instrument and withdrawing the working instrument from the wound.

The method may further include the step of placing an absorbent film on a distal end of the working instrument to be received within the wound. The activation of the working instrument causes an adhesive to be dispensed from the distal end of the working instrument. The activation of the working instrument causes harmonic vibration of the distal end of the working instrument to fuse the wound.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:

FIG. 1 is a perspective view of an embodiment of a wound closure system according to the present disclosure;

FIG. 2 is a side view of the working instrument of the wound closure system of FIG. 1;

FIG. 3A is a distal end view of one embodiment of the working instrument of FIG. 2;

FIG. 3B is a distal end view of an alternate embodiment of the working instrument of FIG. 2;

FIG. 4 is a perspective sectional view of a section of tissue including a puncture wound;

FIGS. 5-9 are progressive cross-sectional side views of the steps of a wound closure procedure using the wound closure system of FIG. 1;

FIG. 10 is a cross-sectional side view of an alternative method of closing a wound according to the present disclosure;

FIG. 11 is a cross-sectional side view of another method of closing a wound according to the present disclosure;

FIG. 12 is an alternative embodiment of a sealing assembly for use in a wound closure system according to the present disclosure; and

FIG. 13 is a cross-sectional side view of the working instrument of the sealing assembly of FIG. 12 fusing tissue.

### DETAILED DESCRIPTION

Embodiments of the presently disclosed wound closure system will now be described in detail with reference to the drawings wherein like numerals designate identical or corresponding elements in each of the several views. As is common in the art, the term "proximal" refers to that part or component closer to the user or operator, i.e. surgeon or physician, while the term "distal" refers to that part or component further away from the user.

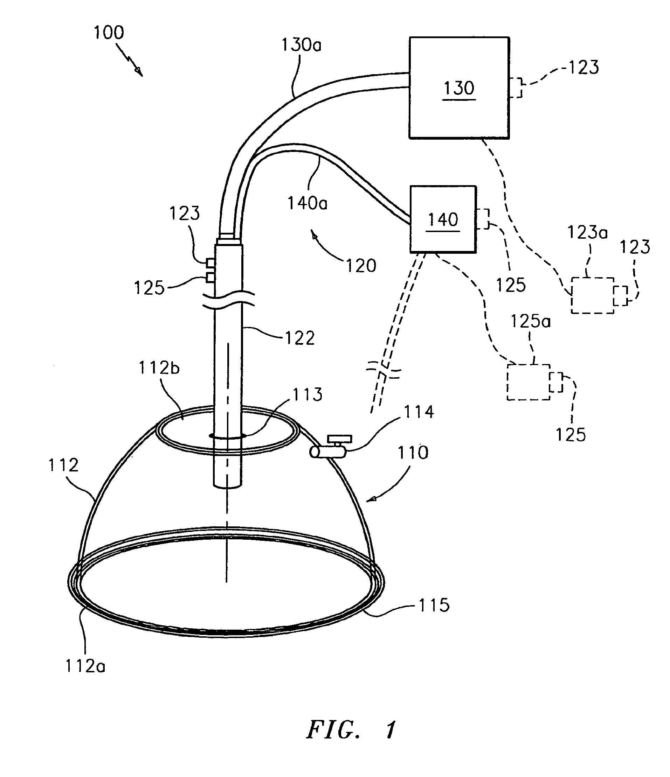

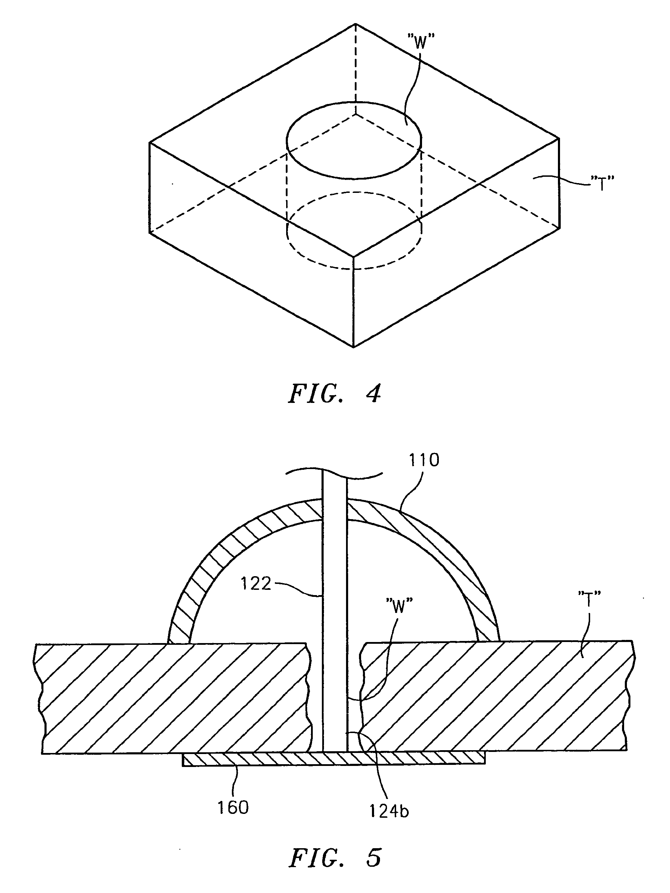

With reference initially to FIG. 1, a system according to an embodiment of the present disclosure is shown generally as wound closure system 100. Wound closure system 100 is configured to seal a wound "W" (FIG. 4) formed in the tissue "T" of a patient. Although wound closure system 100 will be described as used to seal a puncture wound through the abdominal wall of a patient, it is envisioned that wound closure system 100 may be configured for use in closing other types of openings in other locations.

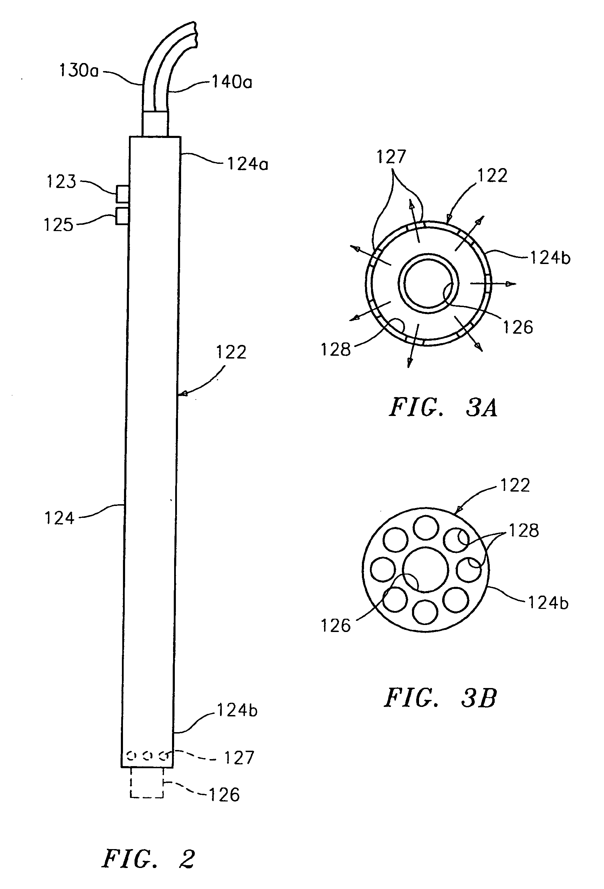

Still referring to FIG. 1, wound closure system 100 includes a housing 110 and a tissue sealing assembly 120. Housing 110 is configured to maintain a vacuum with tissue "T" as a working instrument 122 of sealing assembly 120 is inserted through housing 110 and is operated by the clinician to seal tissue "T". Housing 110 defines a substantially hemi-spherical shaped member 112 including an open tissue contacting end 112a and a substantially closed end 112b. Housing 110 may be of any size and may be formed of plastic, polymer, glass or any other suitable material. As shown, housing 110 is transparent or translucent, however, it is envisioned that housing 110 may instead include a viewing window, or may be configured for use in combination with an imaging device, e.g., x-ray, ultrasound, MRI. Open end 112a of housing 110 is configured to form a seal with tissue "T" (FIG. 3). Open end 112a may include a coating 115 of rubber, silicone or other suitable material for increasing the integrity of the seal between housing 112 and tissue "T". Coating 115 may be applied to open end 112a during manufacture, or instead, may be applied by the clinician prior to use. Open end 112a may be curved, beveled or otherwise configured to match the contour of tissue "T" surrounding wound "W". Substantially closed end 112b includes a slit or other suitable opening 113 configured to receive working instrument 122 of sealing assembly 120 therethrough in a sealed manner.

Still referring to FIG. 1, housing 112 may further include a valve 114 for releasing the vacuum created within housing 112 following completion of the wound closure procedure or in the event the clinician must prematurely removal housing 112. Valve 114 may also be configured for connection with vacuum source 140 for providing vacuum to housing 112. In this manner, and as will be detailed below, vacuum may be created within housing 112 without the use of working instrument 122.

With reference now to FIGS. 1-4, sealing assembly 120 includes working instrument 122 operably connected to an adhesive supply source 130 and vacuum source 140. As shown, adhesive supply source 130 and vacuum source 140 include stand-alone units each in fluid communication with working instrument 122 via tubing 130a, 140a, respectively. It is envisioned however, that adhesive supply source 130 and vacuum source 140 may be integrally formed. In this manner, the tubing connecting the combined unit to working instrument 122 may include a dual lumen. Adhesive supply source 130 may include any device capable of selectively dispensing an adhesive, e.g., a mixing syringe or meter pump. Vacuum source 140 may include any device capable of providing a vacuum to housing 110.

With particular reference now to FIGS. 2-4, working instrument 122 of sealing assembly 120 includes a elongated body 124 having a proximal end 124a, a distal end 124b and defining at least a first and second lumen 126, 128 extending therethrough. Elongated body 124 may be knurled, textured or otherwise configured to facilitate operable engagement by the clinician. Substantially closed proximal end 124a is configured for operable engagement with tubing 130a, 140a. Distal end 124b is configured to be inserted through slit 113 of housing 110 and into a wound "W" (FIG. 5). In this manner, elongated body 24 is sized such that distal end 112b may extend through housing 110 and into wound "W" while proximal end 112a is maintained exterior of housing 112. Distal end 124b of working instrument 122 is further configured for dispensing adhesive and providing vacuum, although, as discussed above, the vacuum may instead be provided directly to housing 112 through valve 114.

Still referring to FIGS. 2-4, first and second lumen 126, 128 are configured to one of, selectively dispense adhesive from adhesive supply source 130 to distal end 124b of working instrument 122 and selectively provide a vacuum from vacuum source 140 to distal end 124b of working instrument 122. Either of lumen 126, 128 may be configured to dispense adhesive or provide a vacuum, depending on the procedure being performed and/or the preference of the clinician performing the procedure. In one embodiment, first and second lumen 126, 128 are coaxially formed within working instrument 122 (FIG. 3A). In an alternate embodiment, first lumen 126 forms a central lumen and second lumen 128 includes a plurality of smaller lumen formed thereabout (FIG. 3B). Either of first and second lumen 126, 128 may extend beyond the other, or otherwise be separated, such that adhesive dispensed from one lumen is not immediately taken into the other lumen. Distal end 124b of working instrument 122 may include any number of openings 127 extending thereabout in fluid communication with one of first or second lumen 126, 128. Although shown as being circular, openings 127 may include any configuration, including rectangular, oval and slotted. In another embodiment, vacuum is provided to housing 110 through valve 114. In this manner, the need for a second lumen within working instrument 122 is eliminated.

With reference to FIGS. 1 and 2, working instrument 122 includes a first switch or valve 123 for activating adhesive supply source 130 and a second switch or valve 125 for activating vacuum source 140. Either or both of first and second switches 123, 125 may be configured to electronically activate adhesive supply source 130 and vacuum source 140, respectively. Alternatively, either or both of first and second switch 123, 125 may be configured to selectively obstruct respective first and second lumens 126, 128 to cause the flow of adhesive 122 or create a vacuum through working instrument 122. In an alternative embodiment, either or both of first and second switch 123, 125 are located on respective adhesive supply source 130 and vacuum source 140 such that they may be activated independently of working instrument 122. In yet another embodiment, either or both of first and second switch 123, 125 are located on a foot pedal 123a, 125a, respectively, for remotely activating respective adhesive supply source 130 and vacuum source 140.

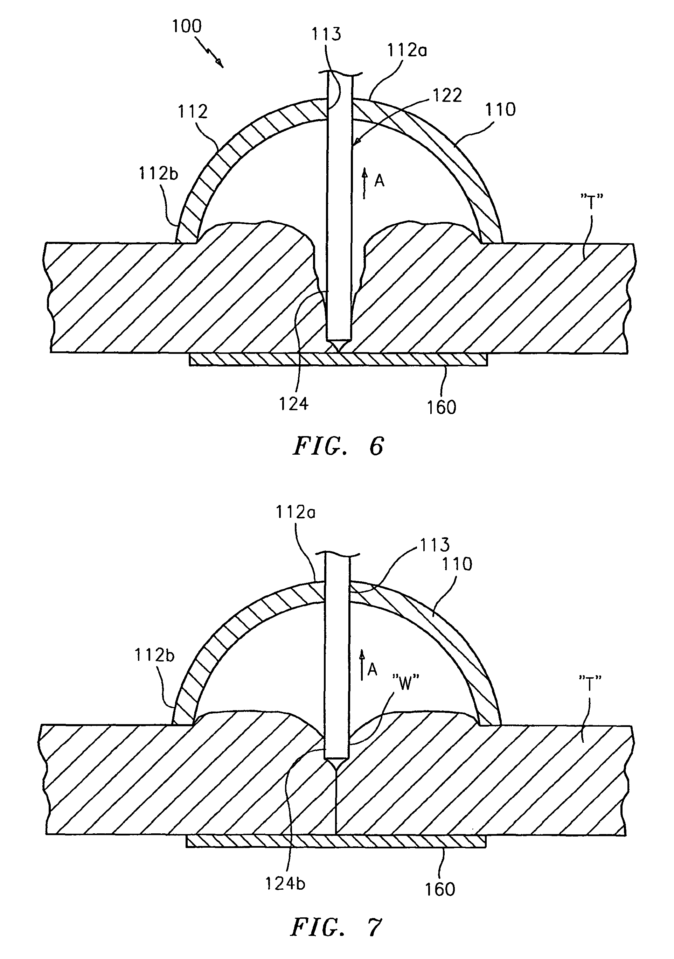

With reference now to FIGS. 5-10, the operation of wound closure system 100 will be described. Initially, a trocar or other access device (not shown) is removed from tissue "T" creating a puncture wound "W" (FIG. 5). Turning to FIG. 6, housing 112 of wound closure system 100 is next placed over wound "W" such that open end 112a of housing 112 engages tissue "T" in an air-tight manner. Distal end 124b of working instrument 122 is then inserted through slit 113 formed in substantially closed distal end 112b of housing 112 and into wound "W". An absorbent film or backing member 160 is releasably affixed to distal end 124b of working instrument 122 prior to insertion into wound "W', or instead may have previously been inserted into wound "W". Absorbent film 160 is included to prevent insufflaction gas from entering wound "W" and breaking the vacuum created through working instrument 122 and/or valve 114. In one embodiment, absorbent film 160 forms a mesh which discourages the sticking of bowels to the film. Distal end 124b of working instrument 122 is inserted through wound "W" such that absorbent film 160 engages an inner surface of tissue "T". Absorbent film 160 may be secured to working instrument 122 with an adhesive or a mechanical fastener, e.g. hook and loop fastener, capable of releasing absorbent film 160 as working instrument 122 is withdrawn from wound "W". In one embodiment, working instrument 122 includes a mechanism (not shown) for separating absorbent film 160 from distal end 124b of working instrument 122.

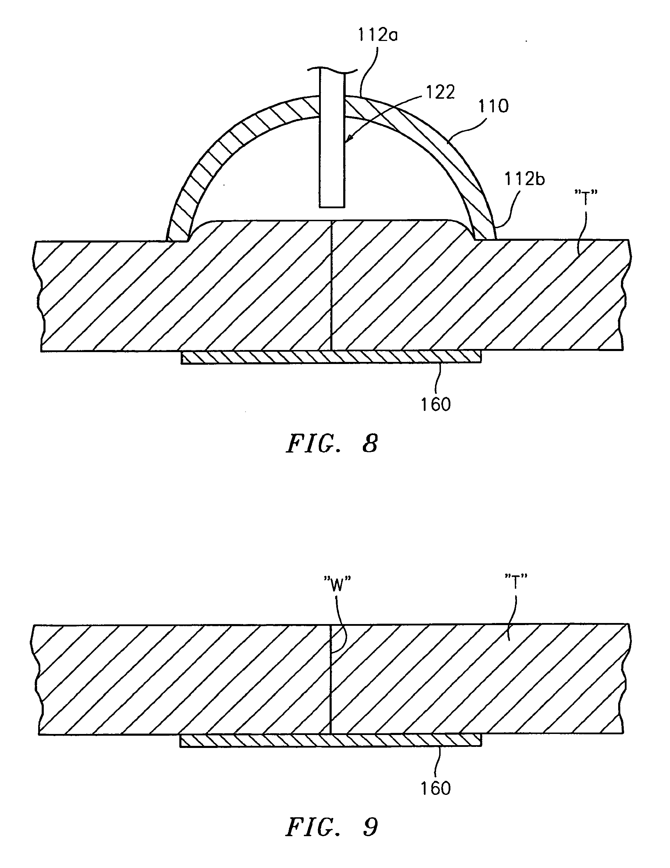

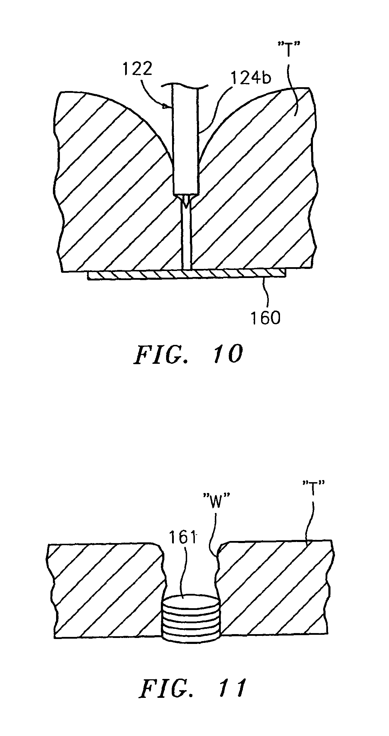

Turning to FIG. 7, once distal end 124b of working instrument 122 and absorbent film 160 have properly been positioned within wound "W", vacuum source 140 is activated to draw the opposed section of tissue "T" about body portion 124 of working instrument 122. Adhesive supply source 130 is then activated to cause adhesive to be dispensed from distal end 124b of working instrument 122. Referring to FIGS. 7 and 8, as distal end 124b of working instrument 122 is retracted from wound "W", in the direction of arrow "A", the vacuum created by vacuum source 140 maintains the opposed section of tissue "T" about distal end 124b of body portion 124 as adhesive is applied to the opposed surfaces of tissue "T". The adhesive applied to tissue "T" may act to bond the opposed surfaces of tissue "T" together (FIG. 9), or instead, the adhesive may be used to form a plug 161 with the void of wound "W" (FIG. 11). Plug 161 may extend completely or partially the length of wound "W". The adhesive used during the wound closure procedure may include any biocompatible adhesives. The adhesive may further be configured to promote wound healing.

With reference now to FIG. 10, once distal end 124b of working instrument 122 is removed from wound "W", adhesive supply source 130 is deactivated. Vacuum source 140 is also deactivated. Alternatively, vacuum source 140 may remain in operation to permit the adhesive time to set. Housing 112 may then be removed from tissue "T" by deactivating vacuum source 140 and releasing the vacuum from within housing 112, either by opening slit 113 or activating valve 114, resulting in a closed wound "W" (FIG. 11).

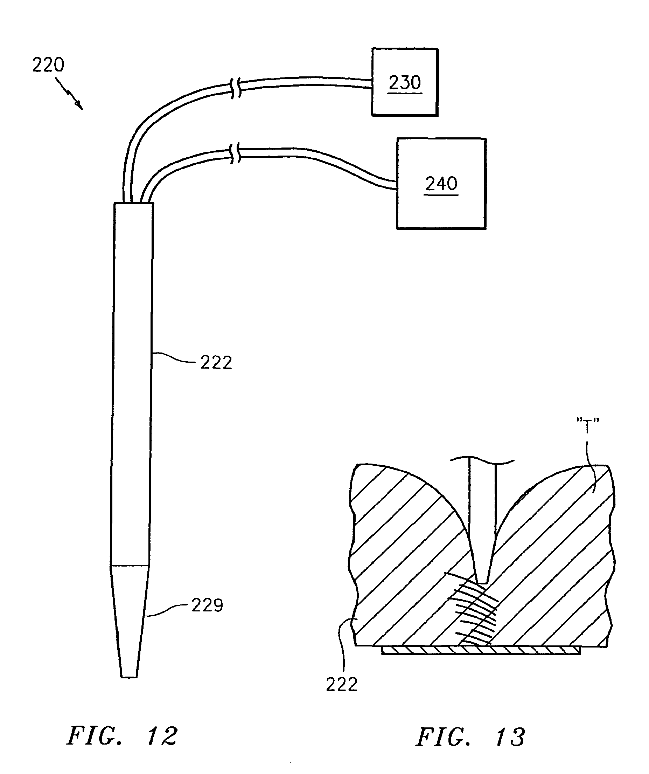

With reference now to FIG. 12, an alternate embodiment of a sealing assembly according to the present disclosure is shown generally as sealing assembly 220. Sealing assembly 220 is substantially similar to sealing assembly 120 and will only be described as relates to the differences therebetween. Sealing assembly 220 includes a working instrument 222 operably connected an electrosurgical generator 230 and a vacuum source 240. Generator 230 provides electrosurgical energy to working instrument 222. A distal end 224b of working instrument includes an extension 229 operably connected to generator 230 and is configured to fuse together opposed surfaces of tissue "T". Extension 229 may be tapered, as shown, or may include any other configuration suitable for directing opposed surfaces of tissue "T" towards working instrument 222. Extension 229 may form a harmonic rod or include an electrode plated member configured to fuse tissue "T" as working instrument 222 is withdrawn from wound "W'. Extension 229 operates to heat tissue "T" surrounding wound "W", thereby fusing tissue "T" and sealing wound "W".

Although the illustrative embodiments of the present disclosure have been described herein with reference to the accompanying drawings, it is to be understood that the disclosure is not limited to those precise embodiments, and that various other changes and modifications may be effected therein by one skilled in the art without departing from the scope or spirit of the disclosure. For example, the working instrument of the tissue sealing assembly may be configured to both dispense adhesive and harmonically seal the tissue.

## Claims

1. A wound closure system comprising:
a housing for maintaining a vacuum about a wound;
a sealing assembly including a working instrument having a distal end configured to be inserted through the housing and into the wound, wherein the working instrument is further configured to close and seal the wound as the working instrument is withdrawn therefrom.

2. The system of claim 1, wherein the housing defines a hemispherical member.

3. The system of claim 1 or claim 2, wherein the housing is transparent.

4. The system of any preceding claim, further including an adhesive supply source operably connected to the working instrument.

5. The system of any preceding claim, further including a vacuum source operably connect to the working instrument.

6. The system of any preceding claim, further including an electrosurgical generator operably connect to the working instrument.

7. The system of any preceding claim, wherein the working instrument further includes at least a first switch for selectively activating a first function of the sealing assembly.

8. The system of claim 7, wherein the working instrument further includes a second switch for selectively activating a second function of the sealing assembly.

9. The system of any preceding claim, wherein the working instrument is configured to selectively dispense an adhesive.

10. The system of any preceding claim, wherein the working instrument is configured to selectively supply a vacuum to the housing.

11. The system of any preceding claim, wherein the working instrument is configured to selectively fuse tissue.

12. The system of any preceding claim, further including an absorbent film for positioning adjacent the wound.

13. A wound closure system according to any preceding claim including a housing and a sealing assembly including a working instrument for use in a method of sealing a wound; wherein the method comprises the steps of:
placing the housing in a sealed relationship over the wound;
inserting a distal end of the working instrument through the housing and into the wound;
applying a vacuum to the housing;
activating the working instrument; and
withdrawing the working instrument from the wound.

14. The wound closure system of claim 13, wherein the method further includes the step of placing an absorbent film on a distal end of the working instrument to be received within the wound.

15. The wound closure system of claim 13, wherein the activation of the working instrument causes an adhesive to be dispensed from the distal end of the working instrument and/or the wound closure system of claim 13, wherein the activation of the working instrument causes harmonic vibration of the distal end of the working instrument to fuse the wound.
